(19) Europäisches Patentamt
European Patent Office
Office européen des brevets

(11) Veröffentlichungsnummer: **0 063 736**
**B1**

(12) # EUROPÄISCHE PATENTSCHRIFT

(45) Veröffentlichungstag der Patentschrift: **08.01.86**

(51) Int. Cl.⁴: **A 61 K 7/13**

(21) Anmeldenummer: **82103092.1**

(22) Anmeldetag: **10.04.82**

(54) **Verwendung von Dihydroxypyridinen als Kupplerkomponente in Oxidationshaarfarbstoffen und Haarfärbemittel.**

(30) Priorität: **18.04.81 DE 3115643**

(43) Veröffentlichungstag der Anmeldung: **03.11.82 Patentblatt 82/44**

(45) Bekanntmachung des Hinweises auf die Patenterteilung: **08.01.86 Patentblatt 86/2**

(84) Benannte Vertragsstaaten: **AT BE CH DE FR GB IT LI NL SE**

(56) Entgegenhaltungen:
**DE - A - 2 629 805**
**DE - A - 2 739 227**

**RECUEIL DES TRAVAUX CHIMIQUES DES PAYS-BAS, Band 63, Nr. 5, Mai 1944, Seiten 231-238, AMSTERDAM (NL). J.P. WIBAUT et al.: "Syntheses of 3,4-Dimethylpyridine, 2,3-Dimethylpyridine and 2-Methyl-3-Ethylpyridine"**
**AUSTRALIAN JOURNAL OF CHEMISTRY, Band 9, Nr. 1, Februar 1956, Seiten 244-251, MELBOURNE (AU). E. RITCHIE: "The Tautomerism of 6-Hydroxy-4-Methyldihydrofuro(2',3',2,3)Pyridine"**
**HELVETIA CHIMICA ACTA, Band 2, Seiten 338-348, 1 919, Basel (CH). L. RUZICKA et al.: "Synthetische Versuche in der Chininreihe I. Synthese des Beta-Collidins"**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Rose, David, Dr., Holbeinweg 7, D-4010 Hilden (DE)**
Erfinder: **Maak, Norbert, Dr., Liebigstrasse 18, D-4040 Neuss (DE)**
Erfinder: **Lieske, Edgar, Hunsrückenstrasse 40, D-4000 Düsseldorf (DE)**

(56) Entgegenhaltungen: (Fortsetzung)
**Die Akte enthält technische Angaben, die nach dem Eingang der Anmeldung eingereicht wurden und die nicht in dieser Patentschrift enthalten sind.**

EP 0 063 736 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der Erfindung ist die Verwendung von substituierten Dihydroxypyridinen als Kupplerkomponenten in Oxidationshaarfarbstoffen. Ein weiterer Gegenstand der Erfindung sind neue Haarfärbemittel auf der Basis von Oxidationsfarbstoffen mit einem Gehalt an substituierten Dihydroxypyridinen als Kupplerkomponenten sowie Haarfärbemittel mit Dihydroxypyridinen als Kuppler- und Tetraaminopyrimidinen als Entwicklerkomponenten.

Für das Färben von Haaren spielen die sogenannten Oxidationsfarbstoffe, die durch oxidative Kupplung einer Entwicklerkomponente und einer Kupplerkomponente entstehen, wegen ihrer intensiven Farben, ihrer unter schonenden Reaktionsbedingungen ablaufenden Bildung und Applikation und ihrer sehr guten Echtheitseigenschaften eine bevorzugte Rolle. Als Entwicklersubstanzen werden üblicherweise Stickstoffbasen wie p-Phenylendiaminderivate, Diaminopyridine, 4-Amino-pyrazolon-derivate oder heterocyclische Hydrazone eingesetzt. Als sogenannte Kupplerkomponenten werden Phenole, Naphthole, Resorcinderivate und Pyrazolone genannt.

Gute Oxidationshaarfarbstoffkomponenten müssen in erster Linie folgende Voraussetzungen erfüllen:

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen, und sie sollen darüber hinaus in toxikologischer und dermatologischer Hinsicht unbedenklich sein. Weiterhin ist von Bedeutung, dass auf dem zu färbenden Haar möglichst kräftige und den natürlichen Haarfarbnuancen weitgehend entsprechende Farbtöne erhalten werden. Ferner kommt der allgemeinen Stabilität der gebildeten Farbstoffe sowie deren Lichtechtheit, Waschechtheit und Thermostabilität erhebliche Bedeutung zu, um Farbverschiebungen von der ursprünglichen Farbnuance oder gar Farbumschläge in andere Farbtöne zu vermeiden. Daneben besteht in der Haarfärberei stets Interesse an neuen Oxidationsfarbstoffkomponenten, die sich mit den bekannten Farbstoffkomponenten zu neuen Farbnuancen von kosmetischem Wert kombinieren lassen.

Aus DE-A 2 629 805 waren Gelbkuppler für Entwicklerkomponenten vom Typ der Tetraaminopyrimidine bekannt, die zur Gruppe der Dihydroxypyridine gehören. Diese erlauben aber in Kombination mit typischen Blaukupplern nicht die Erzeugung gelbgrüner oder olivfarbener Nuancen, wie sie durch Kombination von Gelbkupplern und Blaukupplern entstehen sollten, und eignen sich daher wenig zur Erzeugung natürlicher Haarfärbungen.

Es bestand daher bei der Suche nach neuen, brauchbaren Oxidationshaarfarbstoffen die Aufgabe, geeignete Komponenten aufzufinden, die vorgenannte allgemeine Voraussetzungen in optimaler Weise erfüllen.

Es wurde nun gefunden, dass Dihydroxypyridine der allgemeinen Formel I

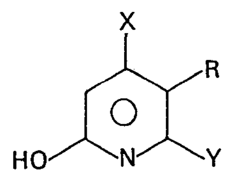

(I)

in welcher R einen kurzkettigen Alkyl- oder Hydroxyalkylrest mit 1–4 Kohlenstoffatomen und X bzw. Y Hydroxylgruppen oder Alkylgruppen mit 1–4 Kohlenstoffatomen darstellen, mit der Massgabe, dass stets einer der Reste X oder Y eine Hydroxylgruppe sein soll, als Kupplerkomponenten in Oxidationshaarfarben den gestellten Anforderungen in besonders hohem Masse gerecht werden.

Bei ihrem Einsatz als Kuppler liefern die erfindungsgemässen Dihydroxypyridine mit den im allgemeinen für die Oxidationshaarfärbung verwendeten Entwicklersubstanzen sehr intensive, von Gelb bis Blaubraun reichende Farbnuancen mit sehr guten Echtheitseigenschaften. Dies gilt in besonderem Masse für die erfindungsgemässen Verbindungen der allgemeinen Formel I, wenn R einen Methyl-, Ethyl- oder 2-Hydroxyethylrest, X (oder Y) eine Hydroxylgruppe und Y (oder X) einen Methylrest darstellt.

Die erfindungsgemässe Verwendung der Dihydroxypyridine als Kupplersubstanzen in Oxidationshaarfarben und Haarfärbemitteln stellt somit eine wesentliche Bereicherung der oxidativen Haarfärbemöglichkeiten dar.

Besonders brillante überwiegend gelbe Farbtöne liefert die erfindungsgemässe Verwendung der Dihydroxypyridine beim Einsatz als Kupplerkomponente in Oxidationshaarfarben, wenn als Entwicklerkomponente eine Verbindung vom Typ der Tetraaminopyrimidin-Derivate der allgemeinen Formel II

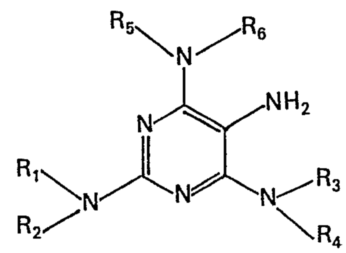

(II)

in der $R_1$–$R_6$ Wasserstoff, einen Alkylrest mit 1–4 Kohlenstoffatomen, den Rest $-(CH_2)_n-X$, in dem $n = 1–4$ und X eine Hydroxylgruppe, ein Halogenatom, eine $-NR'R''$-Gruppe sein können, wobei R′ und R″ Wasserstoff, Alkylreste mit 1–4 Kohlenstoffatomen oder einen den Stickstoff und gegebenenfalls ein weiteres Stickstoff- oder Sauerstoffatom einschliessenden heterocyclischen Ring bedeuten können, und in der $R_1$ und $R_2$, $R_3$ und $R_4$ sowie $R_5$ und $R_6$ mit dem jeweiligen Stickstoffatom zu einem gegebenenfalls ein weiteres Stickstoff- oder Sauerstoffatom einschliessenden heterocyclischen 5- oder 6gliedrigen Ring geschlossen sein können, sowie deren anorganischen oder organischen Salzen, eingesetzt wird.

Das 2.4.5.6-Tetraaminopyrimidin und dessen Abkömmlinge sind als Entwicklerkomponente in Haarfärbemitteln aus der deutschen Patentschrift 2 359 399 bekannt. In Haarfärbemitteln, die diesen Entwickler-Typ verwenden, stellen die erfindungsgemässen Dihydroxypyridine wertvolle Gelbkuppler dar.

In einer besonderen Ausführung der Erfindung werden die erfindungsgemäss zu verwendenden Dihydroxypyridine mit 2.4.5.6-Tetraaminopyrimidin oder dessen Abkömmlingen als Entwickler und 2-Chlor-6-methly-3-aminophenol als Blaukuppler gemeinsam in Haarfärbemitteln eingesetzt. 2-Chlor-6-methyl-3-aminophenol ist ein sehr wertvoller Blaukuppler in Haarfärbemitteln auf Basis von 2.4.5.6-Tetraaminopyrimidinen als Entwickler. In Kombination mit bekannten Gelbkupplern für dieses System, z.B. 2,7-Dihydroxynaphthalin, 6-Hydroxychinolin und 8-Amino-6-methoxychinolin ergeben sich jedoch überwiegend blaustichige Färbungen.

Durch die erfindungsgemässe Verwendung der Dihydroxypyridine als Gelbkuppler in Kombination mit 2-Chlor-6-methyl-3-aminophenol als Blaukuppler werden in Haarfärbemitteln auf Basis von 2.4.5.6-Tetraaminopyrimidin-Abkömmlingen als Entwickler brillante gelbgrüne und olivfarbene Nuancen ohne Blaustich erhalten.

Die erfindungsgemäss als Kupplerkomponenten zu verwendenden Dihydroxypyridine können entweder als solche oder in Form ihrer Salze mit anorganischen oder organischen Säuren, z.B. als Chloride, Sulfate, Phosphate, Acetate, Propionate, Lactate oder Citrate eingesetzt werden. Ausserdem können die erfindungsgemäss zu verwendenden Dihydroxypyridine auch mit weiteren bekannten Kupplerkomponenten gemeinsam in Haarfärbemitteln eingesetzt werden. Beispiele für solche bekannten Kuppler sind z.B. Resorcin, 2-Methylresorcin, 4-Chlorresorcin und 2.4-Dichlor-3-aminophenol.

Die erfindungsgemäss als Kupplerkomponenten zu verwendenden Dihydroxypyridine stellen an sich bekannte Verbindungen dar und lassen sich nach literaturbekannten Verfahren herstellen. So ist z.B. die Herstellung des 2.6-Dihydroxy-3.4-dimethylpyridin sowie des 2.4-Dihydroxy-5.6-dimethylpyridin in Rec. trav. chim. 63 (1944), S. 231, die Herstellung von 2,6-Dihydroxy-3-ethyl-4-methylpyridin in Helv. chim. acta 2, (1918), S. 338, und die Herstellung von 2,6-Dihydroxy-3-β-hydroxyethyl-4-methylpyridin in Austral. J. Chem. 9 (1956), S. 244, beschrieben worden. Die erfindungsgemässe Verwendung der Dihydroxypyridine als Komponente in Haarfärbemitteln ist neu und aus Druckschriften des Standes der Technik nicht zu entnehmen.

Als Beispiele für in den erfindungsgemässen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, p-Aminophenol, N-Methyl-p-phenylendiamin, N,N-Dimethyl-p-phenylendiamin, N,N-Diethyl-2-methyl-p-phenylendiamin, N-Ethyl-N-hydroxyethyl-p-phenylendiamin, Chlorp-phenylendiamin, N,N-Bis-hydroxyethylamino-p-phenylendiamin, Methoxy-p-phenylendiamin, 2,6-Dichlor-p-phenylendiamin, 2-Chlor-6-brom-p-phenylendiamin, 2,5-Diaminoanisol, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1–4 Kohlenstoffatomen darstellt, ferner Diaminopyridinderivate, heterocyclische Hydrazonderivate wie 1-Methylpyrrolidon-(2)-hydrazon, 4-Aminopyrazolonderivate wie 4-Amino-1-phenyl-3-carbamoylpyrazolon-5, N-Butyl-N-sulfobutyl-p-phenylendiamin zu nennen.

Als Beispiele für den vorgenannten Entwickler-Typ der Tetraaminopyrimidine der allgemeinen Formel II, die ebenfalls als solche oder in Form ihrer Salze mit anorganischen oder organischen Säuren, z.B. als Chloride, Sulfate, Phosphate, Acetate, Propionate, Lactate oder Citrate eingesetzt werden können, und die mit grossem Vorteil in Haarfärbemitteln zusammen mit Dihydroxypyridinen erfindungsgemäss verwendet werden, sind folgende Produkte zu nennen:

2,4,5,6-Tetraamino-,
4,5-Diamino-2,6-bismethylamino-,
2,5-Diamino-4,6-bismethylamino-,
4,5-Diamino-6-butylamino-2-dimethylamino-,
2,5-Diamino-4-diethylamino-6-methylamino-,
4,5-Diamino-6-diethylamino--2-dimethylamino-,
4,5-Diamino-2-diethylamino-6-methylamino-,
4,5-Diamino-2-dimethylamino-6-ethylamino-,
4,5-Diamino-2-dimethylamino-6-isopropylamino-,
4,5-Diamino-2-dimethylamino-6-methylamino-,
4,5-Diamino-6-dimethylamino-2-methylamino-,
4,5-Diamino-2-dimethylamino-6-propylamino-,
2,4,5-Triamino-6-dimethylamino-,
4,5,6-Triamino-2-dimethylamino-,
2,4,5-Triamino-6-methylamino-,
4,5,6-Triamino-2-methylamino-,
4,5-Diamino-2-dimethylamino-6-piperidino-,
4,5-Diamino-6-methylamino-2-piperidino-,
2,4,5-Triamino-6-piperidino-,
2,4,5-Triamino-6-anilino-,
2,4,5-Triamino-6-benzylamino-,
2,4,5-Triamino-6-benzylidenamino-,
4,5,6-Triamino-2-piperidino-,
2,4,6-Trismethylamino-5-amino-,
2,4,5-Triamino-6-di-n-propylamino-,
2,4,5-Triamino-6-morpholino-,
2,5,6-Triamino-4-dimethylamino-,
4,5,6-Triamino-2-morpholino-,
2,4,5-Triamino-6-β-hydroxyethylamino-,

4,5,6-Triamino-2-β-amino-ethylamino-,
2,5,6-Triamino-4-β-methylamino-ethylamino-,
2,5-Diamino-4,6-bis-γ-diethylamino-propylamino-,
4,5-Diamino-2-methylamino-6-β-hydroxy-ethylamino-,
5-Amino-2,4,6-triethylamino-,
2,4-Bis-β-hydroxyethylamino-6-anilino-5-aminopyrimidin.

In den erfindungsgemässen Haarfärbemitteln werden die Kupplerkomponenten im allgemeinen in etwa molaren Mengen, bezogen auf die verwendeten Entwicklersubstanzen, eingesetzt. Wenn sich auch der molare Einsatz als zweckmässig erweist, so ist es jedoch nicht nachteilig, wenn die Kupplerkomponente in einem gewissen Überschuss oder Unterschuss zum Einsatz gelangt.

Es ist ferner nicht erforderlich, dass die Entwicklerkomponente und die Kupplersubstanz einheitliche Produkte darstellen, vielmehr können sowohl die Entwicklerkomponente Gemische der erfindungsgemäss zu verwendenden Entwicklerverbindungen als auch die Kupplersubstanz Gemische der erfindungsgemäss einzusetzenden Dihydroxypyridine darstellen.

Darüberhinaus können die erfindungsgemässen Haarfärbemittel gegebenenfalls übliche direkt ziehende Farbstoffe im Gemisch enthalten, falls dies zur Erzielung gewisser Farbnuancen erforderlich ist.

Die oxidative Kupplung, d.h. die Entwicklung der Färbung, kann grundsätzlich wie bei anderen Oxidationshaarfarbstoffen auch durch Luftsauerstoff erfolgen. Zweckmässigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Als Entwicklerkomponente besitzen dabei die Tetraaminopyrimidine den Vorteil, dass sie bereits bei oxidativer Kupplung durch Luftsauerstoff voll befriedigende Färbeergebnisse liefern und somit eine Haarschädigung durch das sonst für die oxidative Kupplung eingesetzte Oxidationsmittel vermieden werden kann. Wird jedoch gleichzeitig neben der Färbung ein Aufhelleffekt am Haar gewünscht, so ist die Mitverwendung von Oxidationsmitteln erforderlich.

Die erfindungsgemässen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind z.B. Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie z.B. Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemässen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 bis 10 erfolgen. Die Anwendungstemperaturen bewegen sich dabei im Bereich von 15 bis 40°C. Nach einer Einwirkungsdauer von ca. 30 Minuten wird das Haarfärbemittel vom zu färbenden Haar durch Spülen entfernt. Hernach wird das Haar mit einem milden Shampoo nachgewaschen und getrocknet.

Die nachfolgenden Beispiele sollen den Erfindungsgegenstand näher erläutern, ohne ihn jedoch hierauf zu beschränken.

Beispiele

Als erfindungsgemäss zu verwendende Kupplerkomponenten wurden die folgenden als Beispiele in Oxidationshaarfarben bzw. Haarfärbemitteln eingesetzt:

K 1: 2,6-Dihydroxy-3,4-dimethylpyridin
K 2: 2,4-Dihydroxy-5,6-dimethylpyridin
K 3: 2,6-Dihydroxy-3-ethyl-4-methylpyridin
K 4: 2,6-Dihydroxy-3-(2-hydroxyethyl)-4-methylpyridin

Als Entwicklerkomponenten dienten die folgenden Substanzen:

E 1: p-Toluylendiamin
E 2: p-Phenylendiamin
E 3: 2-Chlor-p-phenylendiamin
E 4: 2,5-Diaminoanisol
E 5: N,N-Bis-2-hydroxyethyl-p-phenylendiamin
E 6: N-2-Methoxyethyl-p-phenylendiamin
E 7: N-2-Hydroxyethyl-p-phenylendiamin
E 8: N-2-Hydroxypropyl-p-phenylendiamin
E 9: N-Methyl-p-phenylendiamin
E 10: N-Butyl-N-sulfobutyl-p-phenylendiamin
E 11: 2,4,5,6-Tetraaminopyrimidin
E 12: 2-Piperidino-4,5,6-triaminopyrimidin
E 13: 2-Morpholino-4,5,6-triaminopyrimidin

Die erfindungsgemässen Haarfärbemittel wurden in Form einer Creme-Emulsion eingesetzt. Dabei wurden in eine Emulsion aus

10 Gewichtsteilen Fettalkoholen der Kettenlänge $C_{12}$–$C_{18}$
10 Gewichtsteilen Fettalkoholsulfat (Natriumsalz) der Kettenlänge $C_{12}$–$C_{18}$
75 Gewichtsteilen Wasser
jeweils 0,01 Mol der in der nachstehenden Tabelle 1 aufgeführten Entwicklersubstanzen und 0,01 Mol der aufgeführten Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 3%iger Wasserstoffperoxidlösung als Oxidationsmittel durchgeführt, wobei zu 100 Gewichtsteilen der Emulsion 10 Gewichtsteile Wasserstoffperoxidlösung gegeben wurde.

Nach Zusatz des Oxidationsmittels wurde die jeweilige Färbecreme auf standardisiertes, zu 90% ergrautes, nicht besonders vorbehandeltes Menschenhaar aufgetragen und dort 30 Minuten bei Umgebungstemperatur belassen. Nach Beendigung des Färbeprozesses wurde das Haar mit einem handelsüblichen Haarwaschmittel ausgewaschen und anschliessend getrocknet. Die dabei erhaltenen Färbungen sind aus Tabelle 1 zu entnehmen:

Tabelle 1

| Beispiel | Entwickler | Kuppler | Farbton des gefärbten Haares nach Oxidation mit 3%iger $H_2O_2$-Lösung |
|---|---|---|---|
| 1 | E 1 | K1 | violettbraun |
| 2 | E 2 | K1 | violettgrau |
| 3 | E 3 | K1 | rotbraun |
| 4 | E 4 | K1 | nutria |
| 5 | E 5 | K1 | blaugrau |
| 6 | E 6 | K1 | olivgrau |
| 7 | E 7 | K1 | grüngrau |
| 8 | E 8 | K1 | oliv |
| 9 | E 9 | K1 | grüngrau |
| 10 | E 10 | K1 | grüngrau |
| 11 | E 11 | K1 | gelb |
| 12 | E 12 | K1 | gelb |
| 13 | E 13 | K1 | gelb |
| 14 | E 11 | K2 | sandgelb |
| 15 | E 11 | K3 | kanariengelb |
| 16 | E 11 | K4 | gelb |
| 17 | E 1 | K2 | aubergine |

Die besondere Eignung der erfindungsgemässen Verwendung der Dihydroxypyridine als Gelbkupplerkomponente in Oxidations-Haarfärbemitteln mit 2,4,5,6-Tetraaminopyrimidin-Derivaten als Entwicklerkomponente wird aus den Beispielen Nr. 11–16 ersichtlich.

Die besondere Eignung der erfindungsgemässen Verwendung der Dihydroxypyridine als Gelbkupplerkomponente gemeinsam mit 2-Chlor-6-methyl-3-aminophenol-hydrochlorid (K8) als Blaukuppler in Oxidations-Haarfärbemitteln mit 2,4,5,6-Tetraaminopyrimidin-Derivaten als Entwicklerkomponente soll durch folgende Beispiele Nr. 18–29 in Tabelle 2 gezeigt werden.

2-Chlor-6-methyl-3-aminophenol-hydrochlorid (K8) wurde nach folgender Methode erhalten:

14 g 2-Chlor-6-methyl-3-nitrophenol, welches gemäss den Angaben in den Annalen der Chemie 417 (1918), Seite 246, hergestellt worden war, wurde in 50 ml Ethanol in Gegenwart von Raney-Nickel katalytisch hydriert. Nach beendeter Wasserstoffaufnahme wurde der Katalysator abfiltriert. Die Lösung wurde mit Salzsäure angesäuert und zur Trockne eingeengt. Dabei wird das 2-Chlor-6-methyl-3-aminophenol-hydrochlorid in Form weisser Kristalle vom Schmelzpunkt 163°C erhalten.

Für Vergleichszwecke wurden die folgenden, allgemein bekannten Kupplerkomponenten, die mit 2,4,5,6-Tetraaminopyrimidin als Entwickler gelbe Färbungen ergeben, eingesetzt.

K 5: 2,7-Dihydroxynaphthalin
K 6: 6-Hydroxychinolin
K 7: 8-Amino-methoxychinolin.

Die Vergleichsversuche wurden mit einer Färbecreme-Emulsion der folgenden Zusammensetzung durchgeführt:

| | | |
|---|---|---|
| 10,2 | g | Talgfettalkohol der Kettenlänge $C_{16}$-$C_{18}$ |
| 2,4 | g | Kokosfettalkohol der Kettenlänge $C_{12}$–$C_{18}$ |
| 30,6 | g | Fettalkohol $C_{12-14}$-diglykolethersulfat, Natriumsalz (28 Gew.%ige Lösung, Texapon N 25®, Henkel KGaA) |
| 0,0025 | Mol | 2,4,5,6-Tetraaminopyrimidinsulfat |
| 0,0025 | Mol | Kupplerkombination (s. Tabelle 2) |
| 57 | g | Wasser und Ammoniak bis pH 9,5. |

Die Herstellung der Färbecreme, die oxidative

Kupplung mit 3%iger Wasserstoffperoxidlösung sowie die Ausfärbung des standardisierten, zu 90% ergrauten Haares und das Auswaschen des Haares mit einem handelsüblichen Haarwaschmittel erfolgte wie zu den Beispielen 1–17 beschrieben.

Als Kupplerkombination wurden Mischungen von 2-Chlor-6-methyl-3-aminophenol-hydrochlorid (K8) und bekannten Gelbkupplern K5–K7 sowie dem neuen Gelbkuppler K1 in unterschiedlichen molaren Mischungsverhältnissen eingesetzt.

Tabelle 2

| Beispiel | Kuppler-kombination | molares Mischungs-verhältnis | Farbton des gefärbten Haares nach Oxidation mit 3%iger $H_2O_2$-Lösung |
|---|---|---|---|
| 18 | K8 : K5 | 7 : 3 | blau |
| 19 | K8 : K5 | 5 : 5 | blau |
| 20 | K8 : K5 | 3 : 7 | blaugrau |
| 21 | K8 : K6 | 7 : 3 | blau |
| 22 | K8 : K6 | 5 : 5 | blau |
| 23 | K8 : K6 | 3 : 7 | graugrün |
| 24 | K8 : K7 | 7 : 3 | blau |
| 25 | K8 : K7 | 5 : 5 | blau |
| 26 | K8 : K7 | 3 : 7 | olivgrau |
| 27 | K8 : K1 | 7 : 3 | türkis |
| 28 | K8 : K1 | 5 : 5 | oliv |
| 29 | K8 : K1 | 3 : 7 | gelbgrün |

**Patentansprüche**

1. Verwendung von Dihydroxypyridinen der allgemeinen Formel I

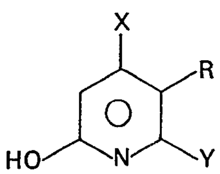

(I)

in welcher R einen kurzkettigen Alkyl- oder Hydroxyalkylrest mit 1–4 Kohlenstoffatomen und X bzw. Y eine Hydroxylgruppe oder eine Alkylgruppe mit 1–4 Kohlenstoffatomen darstellen, mit der Massgabe, dass einer der Reste, X oder Y, eine Hydroxylgruppe sein soll, sowie deren Salzen mit anorganischen oder organischen Säuren, als Kupplerkomponenten in Oxidationshaarfarbstoffen.

2. Verwendung von Dihydroxypyridinen der allgemeinen Formel I, in welcher R einen Methyl-, Ethyl- oder 2-Hydroxyethylrest darstellt und X bzw. Y eine Hydroxylgruppe oder einen Methylrest darstellt, mit der Massgabe, dass einer der Reste, X oder Y, eine Hydroxylgruppe sein soll, sowie deren Salzen mit anorganischen oder organischen Säuren, als Kupplerkomponenten in Oxidationshaarfarbstoffen.

3. Haarfärbemittel auf Basis von Oxidationsfarbstoffen mit einem Gehalt an Dihydroxypyridinen gemäss Anspruch 1 und 2 als Kupplerkomponenten sowie gegebenenfalls weiteren üblichen Kupplersubstanzen und den in Oxidationsfarben üblichen Entwicklerkomponenten sowie gegebenenfalls üblichen direktziehenden Farbstoffen.

4. Haarfärbemittel nach Anspruch 3, dadurch gekennzeichnet, dass als Entwicklerkomponente Tetraaminopyrimidine der allgemeinen Formel II

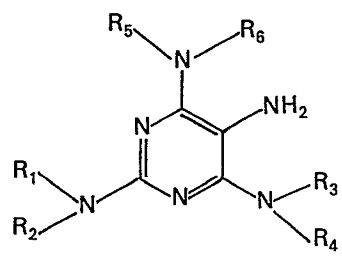

(II)

in der $R^1$–$R^6$ Wasserstoff, einen Alkylrest mit 1–4 Kohlenstoffatomen, den Rest $-(CH_2)_n-X$, in dem n = 1–4 und X eine Hydroxylgruppe, ein Halogenatom, eine $-NR'R''$-Gruppe, wobei R′ und R″ Wasserstoff oder Alkylreste mit 1–4 Kohlenstoffatomen bedeuten können oder mit dem Stickstoffatom zu einem gegebenenfalls ein weiteres Stickstoff- oder Sauerstoffatom enthaltenden heterocyclischen Ring geschlossen sind, bedeuten, oder $R^1$ und $R^2$ bzw. $R^3$ und $R^4$ bzw. $R^5$ und $R^6$ mit dem jeweiligen Stickstoffatom einen heterocyclischen, 5- oder 6gliedrigen Ring mit einem oder zwei Stickstoffatomen oder einem Stickstoff- und einem Sauerstoffatom bilden können, sowie deren Salze mit anorganischen oder organischen Säuren, verwendet werden.

5. Haarfärbemittel nach Anspruch 4, dadurch gekennzeichnet, dass als weitere Kupplerkomponente 2-Chlor-6-methyl-3-aminophenol oder dessen anorganisches oder organisches Salz enthalten ist.

6. Haarfärbemittel nach einem der Ansprüche 3–5, gekennzeichnet durch einen Gehalt an Entwickler/Kuppler-Kombination von 0,2–5,0 Gewichtsprozent, vorzugsweise 1–3 Gewichtsprozent, bezogen auf das gesamte Haarfärbemittel.

**Claims**

1. The use of dihydroxypyridines corresponding to the following general formula

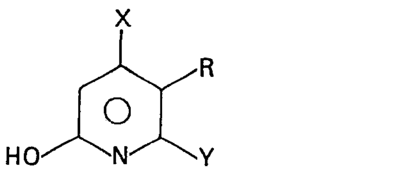

(I)

in which R represents a short-chain alkyl or hydroxyalkyl group containing from 1 to 4 carbon atoms and X and Y represent a hydroxyl group or an alkyl group containing from 1 to 4 carbon atoms, with the proviso that either X or Y must be a hydroxyl group, and salts thereof with inorganic or organic acids as coupler components in oxidation hair dyes.

2. The use of dihydroxypyridines corresponding to general formula I in which R is a methyl, ethyl or 2-hydroxyethyl group and X and Y represent a hydroxyl group or a methyl group, with the proviso that either X or Y must be a hydroxyl group, and salts thereof with inorganic or organic acids as coupler components in oxidation hair dyes.

3. Hair dyes based on oxidation dyes containing the dihydroxypyridines claimed in Claims 1 and 2 as coupler components and, optionally, other standard couplers, the developer components normally used in oxidation dyes and, optionally, standard substantive dyes.

4. Hair dyes as claimed in Claim 3, characterized in that tetra-aminopyridines corresponding to the following general formula

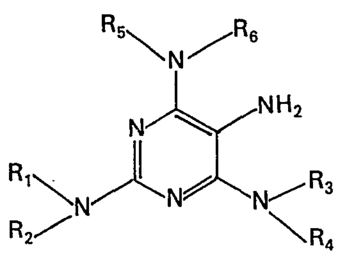

(II)

in which $R^1$ to $R^6$ represent hydrogen, a $C_1$–$C_4$ alkyl group, the group $-(CH_2)_n-X$ in which $n = 1$–$4$ and X is a hydroxyl group, a halogen atom, an $-NR'R''$-group wherein R′ and R″ may represent hydrogen or $C_1$–$C_4$ alkyl groups or are closed with the nitrogen atom to form a heterocyclic ring optionally containing another nitrogen or oxygen atom, or $R^1$ and $R^2$ or $R^3$ and $R^4$ or $R^5$ and $R^6$ may form with the associated nitrogen atom a heterocyclic 5- or 6-membered ring containing one or two nitrogen atoms or one nitrogen and one oxygen atom, and salts thereof with inorganic or organic acids are used as developer component.

5. Hair dyes as claimed in Claim 4, characterized in that 2-chloro-6-methyl-3-aminophenol or an inorganic or organic salt thereof is present as further coupler component.

6. Hair dyes as claimed in any of Claims 3 to 5, characterized by a content of developer-coupler component of from 0.2 to 5.0% by weight and preferably from 1 to 3% by weight, based on the hair dye as a whole.

**Revendications**

1. Utilisation de dihydroxypyridines de formule générale I:

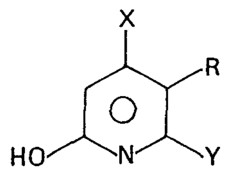

(I)

dans laquelle R représente un radical alcoyle ou hydroxyalcoyle à chaîne courte ayant 1 à 4 atomes de carbone et X ou Y un groupe hydroxyle ou un groupe alcoyle ayant 1 à 4 atomes de carbone, avec comme condition qu'un des radicaux, X ou Y, doit être un groupe hydroxyle, de même que de leurs sels avec des acides minéraux ou organiques, comme composants coupleurs dans des colorants d'oxydation pour cheveux.

2. Utilisation de dihydroxypyridines de formule générale I, dans laquelle R représente un radical méthyle, éthyle ou 2-hydroxyéthyle et X ou Y un groupe hydroxyle ou un radical méthyle, avec comme condition qu'un des radicaux, X ou Y, doit être un groupe hydroxyle, ainsi que leurs sels avec des acides minéraux ou organiques, comme composants de couplage dans des colorants d'oxydation pour cheveux.

3. Agent de teinture pour cheveux à base de colorants d'oxydation ayant une teneur en dihydroxypyridines selon les revendications 1 et 2 comme composants coupleurs, de même qu'éventuellement en d'autres substances usuelles de couplage, et en des composants de développement usuels dans des teintures oxydantes, de même qu'éventuellement en des colorants montants directs usuels.

4. Agent de teinture pour cheveux selon la revendication 3, caractérisé en ce qu'on utilise comme composant de développement des tétraaminopyrimidines de formule générale II:

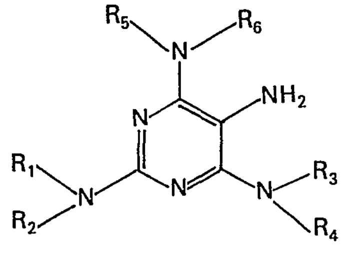

(II)

dans laquelle $R^1$ à $R^6$ peuvent signifier de l'hydrogène, un radical alcoyle ayant 1 à 4 atomes de carbone, le radical $-(CH_2)_n-X$ dans lequel $n = 1$ à 4 et X un groupe hydroxyle, un atome d'halogène, un groupe $-NR'R''$ avec R′ et R″ qui sont de

l'hydrogène ou des radicaux alcoyle ayant 1 à 4 atomes de carbone, ou signifient par cyclisation avec l'atome d'azote un noyau hétérocyclique contenant éventuellement un atome supplémentaire d'azote ou d'oxygène, ou bien $R^1$ et $R^2$, ou $R^3$ et $R^4$, ou $R^5$ et $R^6$ peuvent former avec l'atome d'azote chaque fois concerné un noyau hétérocyclique à 5 ou 6 chaînons, avec un ou deux atomes d'azote ou un atome d'azote et un atome d'oxygène, de même que leurs sels avec des acides minéraux ou organiques.

5. Agent de teinture pour cheveux selon la revendication 4, caractérisé en ce qu'il contient comme composant coupleur supplémentaire du 2-chloro-6-méthyl-3-aminophénol ou un sel minéral ou organique de ce dernier.

6. Agent de teinture pour cheveux selon l'une des revendications 3 à 5, caractérisé par une teneur en une combinaison agent de développement-coupleur de 0,2 à 5,0% en poids, de préférence de 1 à 3% en poids par rapport à l'agent de teinture pour cheveux total.